# EUROPEAN PATENT APPLICATION

(11) **EP 4 234 567 A1**
(43) Date of publication of application: **30.08.2023**
(21) Application number: 21883209.5
(22) Date of filing: 19.10.2021
(51) Int. Cl.: C07H 21/02, C12N 15/113, A61K 31/7088

(54) **OLIGONUCLEOTIDE FOR 5'-CAPPED RNA SYNTHESIS**

(30) Priority: 20.10.2020 KR 20200135823
(71) Applicant: ST Pharm Co., Ltd., Siheung-si, Gyeonggi-do 15086 (KR)
(72) Inventor: KIM, Kyung Jin, Seoul 06194 (KR); CHOI, Kang Hyun, Ansan-si, Gyeonggi-do 15610 (KR); KIM, Uk-Il, Ansan-si, Gyeonggi-do 15610 (KR); BANG, Hyung Tae, Ansan-si, Gyeonggi-do 15610 (KR); LEE, Seul Ki, Ansan-si, Gyeonggi-do 15610 (KR); HAN, Si Yeon, Ansan-si, Gyeonggi-do 15610 (KR)
(74) Representative: D Young & Co LLP
(86) International application number: PCT/KR2021/014625
(87) International publication number: WO 2022/086140

(57) **Abstract**

The present invention relates to a novel oligonucleotide primer used for the synthesis of 5'-capped RNA. The novel oligonucleotide primer for RNA capping provided as Formula 1 in the present invention can be usefully utilized in the fields of nucleic acid therapeutic agents or vaccines.

## Description

### [Technical Field]

The present invention relates to a novel oligonucleotide used for the synthesis of 5'-capped RNA.

### [Background Art]

The 5'-capping of RNA is the first step in the premRNA processing process in the eukaryotic cells, which is a process in which a cap is formed on the 5' end of a transcribed RNA. On the 5' end of mRNA molecule in eukaryotic organism, 5' cap (cap-0) connected with 7-methylguanosine (m⁷G) by 5' triphosphate linkage is formed, which is mediated by a methyltransferase in vivo. The mRNA of the eukaryotic organism may have additional variations including the methylation (cap-1) of the 2' hydroxyl group of the first ribose sugar and the methylation (cap-2) of the 2' hydroxyl group of the second ribose sugar of 5'-end. This 5' capping process of mRNA provides resistance to degradation enzymes such as 5' exonuclease when mRNA exits from nucleus to cytoplasm.

For medical or research purposes, the gene may be artificially expressed by transfecting the capped mRNA that codes a particular gene into the eukaryotic organism or microinjecting the mRNA into cells or embryos. However, when uncapped RNA is used in this process, RNA is rapidly degraded as well as has immunogenicity and drastically reduced protein translation efficiency. Accordingly, it is very important that 5' capping is performed properly to synthesize mRNA in vitro.

For mRNA synthesis, it has been found that a process in which RNA having a 5'-triphosphate group is first transcribed in vitro, followed by 5' capping using a capping enzyme, is not only expensive but also inefficient. Accordingly, a synthetic method comprising producing an oligonucleotide having a 5'-capped structure and initiating in vitro transcription using the oligonucleotide as a primer has been developed. For example, International Publication WO2008/016473 and International Publication WO2013/059475 disclose dinucleotide mRNA cap analogs for synthesizing 5'-capped RNA, and International Publication WO2017/053297 discloses trinucleotide mRNA cap analogues for synthesizing 5'-capped RNA.

Since the capped RNA transcripts are able to be applied to therapeutic and/or preventive fields requiring protein synthesis, such as nucleic acid therapeutic agents and vaccines, it is necessary to develop an oligonucleotide primer for 5'-capping for efficiently producing the RNA transcripts. There is an urgent need for the development of RNA cap analogues, for example, capable of being manufactured more cost-effectively with simpler manufacturing process than conventional methods, efficiently performing a desired transcription reaction, improving RNA production yield, reducing the production of unwanted heterogeneous products, not requiring additional enzymatic reactions, or enabling large-scale synthesis of RNA. In addition, if a cap structure is provided that is able to maintain mRNA stability in vivo, improve translation efficiency, or reduce side effects such as immunogenicity, and the like, when applied as an actual RNA therapeutic agent or vaccine, it is possible to achieve universal utility in the field of RNA therapeutic agents or vaccines.

### [Detailed Description]

### [Technical Problem]

An object of the present invention is to provide a novel oligonucleotide primer for RNA capping, a method for preparing the same, and use thereof.

Another object of the present invention is to provide an RNA molecule prepared using the oligonucleotide primer for RNA capping and use thereof.

### [Technical Solution]

The present inventors provide a novel oligonucleotide primer for RNA capping, a method for preparing the same, and a method for using the same. Unless defined otherwise, all terms used herein have the same meaning as commonly understood by a person skilled in the art to which this invention belongs. All patent and non-patent documents described throughout the disclosure herein are incorporated by reference in their entirety.

### Novel oligonucleotide primer for RNA capping

The present invention provides a compound represented by the following Formula 1 or a stereoisomer thereof, or a salt thereof:

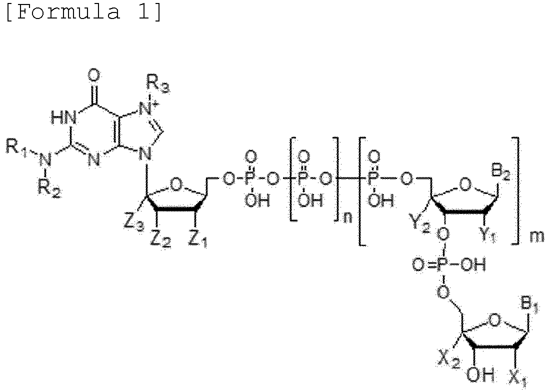

in Formula 1 above,
B₁ and B₂ are each independently a natural, unnatural or modified nucleoside base;
X₁ is -OH, -O(C₁₋₄alkyl), or -halo;
X₂ is -H or linked with X₁ to form an LNA ring {wherein at least one H of the LNA ring may be substituted with -(C₁₋₄alkyl), -OH, or -O(C₁₋₄alkyl)};
Y₁ is -O(C₁₋₄alkyl), -O(C₁₋₄alkyl)O(C₁₋₄alkyl), -CH₂O(C₁₋₄alkyl), or -halo;
Y₂ is -H or linked with Y₁ to form an LNA ring {wherein at least one H of the LNA ring may be substituted with -(C₁₋₄alkyl), -OH, or -O(C₁₋₄alkyl)};
Z₁ and Z₂ are each independently -OH, -O(C₁₋₄alkyl), - O(C₁₋₄alkyl)O(C₁₋₄alkyl), -CH₂O(C₁₋₄alkyl), or -halo;
Z₃ is -H or linked with Z₁ to form an LNA ring {wherein at least one H of the LNA ring may be substituted with -(C₁₋₄alkyl), -OH, or -O(C₁₋₄alkyl)};
n is an integer from 0 to 3;
m is an integer from 1 to 10 {wherein when m is 2 or more, each of B₂, Z₁ and Z₂ may be different from each other};
R₁ and R₂ are each independently -H, or -(C₁₋₄alkyl); and
R₃ is -(C₁₋₄alkyl).

In the present invention, as the nucleoside base, natural nucleoside bases as well as non-natural or modified nucleoside bases may be used.

In the present invention, base rings most commonly found in naturally occurring nucleosides are purine and pyrimidine rings. Naturally occurring purine rings include, for example, adenine 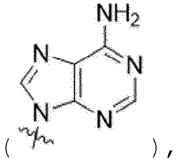 guanine 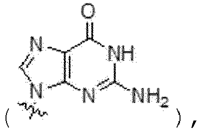 and N⁶-methyladenine 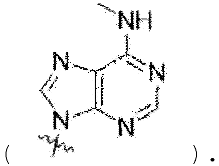 Naturally occurring pyrimidine rings include, for example, cytosine 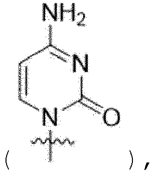 thymine 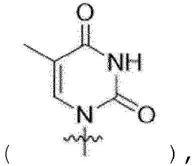 5-methylcytosine 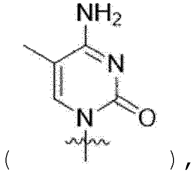 and uracil

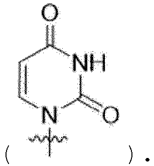

According to an embodiment of the present invention, the unnatural or modified nucleoside base may be an isomer of the natural nucleoside base or may be substituted with at least one selected from the group consisting of substitution of at least one -H of the natural nucleoside base with -(C₁₋₄alkyl), -O(C₁₋₄alkyl) or -halo, substitution of at least one =CH- with =N-, and substitution of at least one =O with =S.

Further, in the present invention, the unnatural or modified nucleoside bases may be, as a substitute for one of the natural NTPs (for example, ATP, UTP, CTP and GTP) or other specific NTPs, bases derived from nucleoside analogs with artificial bases recognizable by RNA polymerase (see, Loakes, D., Nucleic Acids Res., 29:2437-2447 (2001); Crey-Desbiolles, C., et al., Nucleic Acids Res., 33:1532-1543 (2005); Kincaid, K., et al., Nucleic Acids Res., 33:2620-2628 (2005); Preparata, FP, Oliver, JS, J. Comput. Biol. 753-765 (2004); and Hill, F., et al., Proc Natl Acad. Sci. USA, 95:4258-4263 (1998)], and the like).

In addition, in the present invention, the unnatural or modified nucleoside base may be a base derived from halogen-substituted purine (for example, 6-fluoropurine), halogen-substituted pyrimidine, N⁶-ethyladenine, N⁴-(alkyl)-cytosine, 5-ethylcytosine, and the like, but is not limited thereto.

In addition, in the present invention, as another example of the non-natural or modified nucleoside base, 8-azaguanosine, pseudouridine (ψ), 5-methyl-cytidine (m⁵C), 1-methyl-pseudouridine (m¹ψ), 1-methyl-pseudouridine (m¹ψ) and 5-methyl-cytidine (m⁵C), 2-thiouridine (s²U), 2-thiouridine (s²U) and 5-methyl-cytidine (m⁵C), 5-methoxy-uridine (mo⁵U), 5-methoxy-uridine (mo⁵U) and 5-methyl-cytidine (m⁵C), 2'-O-methyl uridine, 2'-O-methyl uridine and 5-methyl-cytidine (m⁵C), N⁶-methyl-adenosine (m⁶A) or N⁶-methyl-adenosine (m⁶A) and 5-methyl-cytidine (m⁵C) may be included, but the non-natural or modified nucleoside base is not limited thereto.

In addition thereto, examples of the natural, non-natural or modified nucleoside bases capable of being used in the present invention may be referred to in documents (see International Publication WO2018/144775, International Publication WO2018/144082, and the like).

According to an embodiment of the present invention, B₁ and B₂ may each independently be

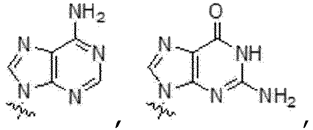

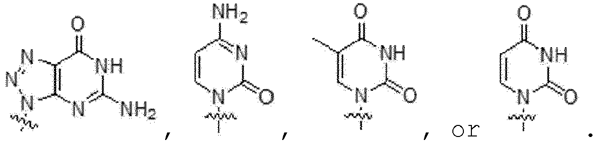

In addition, according to an embodiment of the present invention,
X₁ may be -OH; and
X₂ may be -H.

In addition, according to an embodiment of the present invention,
Y₁ may be -O(C₁₋₄alkyl), or -O(C₁₋₄alkyl)O(C₁₋₄alkyl), or -halo; and
Y₂ may be -H or linked with Y₁ to form an LNA ring {wherein at least one H of the LNA ring may be substituted with - (C₁₋₄alkyl)} .

In addition, according to an embodiment of the present invention,
Z₁ may be -OH, or -O(C₁₋₄alkyl);
Z₂ may be -OH; and
Z₃ may be -H.

Further, according to an embodiment of the present invention, n may be 0 or 1.

Further, according to an embodiment of the present invention, m may be 1.

In addition, according to an embodiment of the present invention,
R₁ and R₂ may be each independently -H; and
R₃ may be - (C₁₋₄alkyl).

Specifically, the compound represented by Formula 1 may be any one selected from the group consisting of the following compounds: 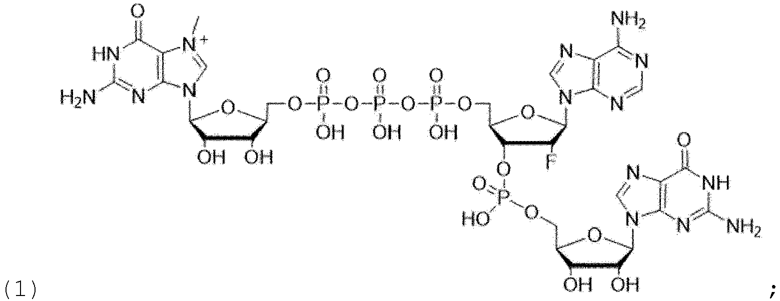 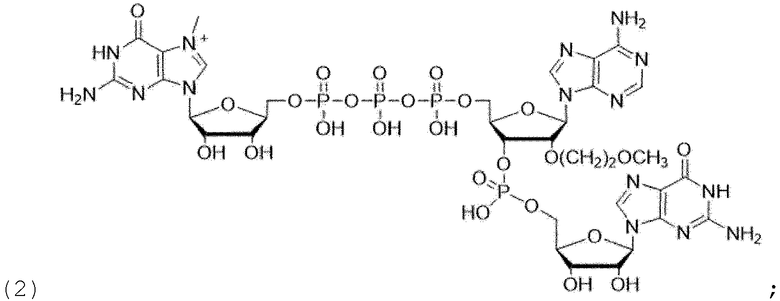 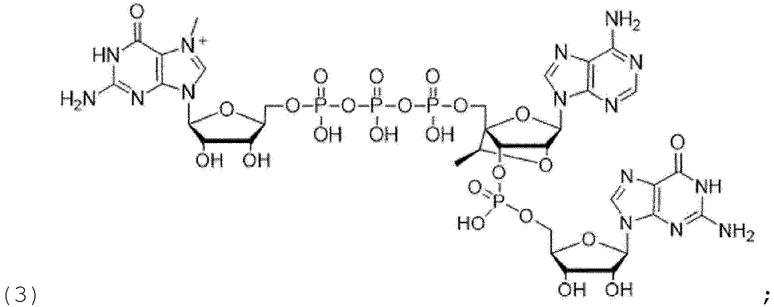 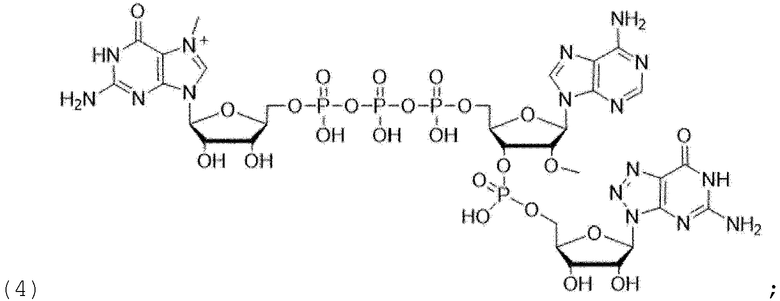 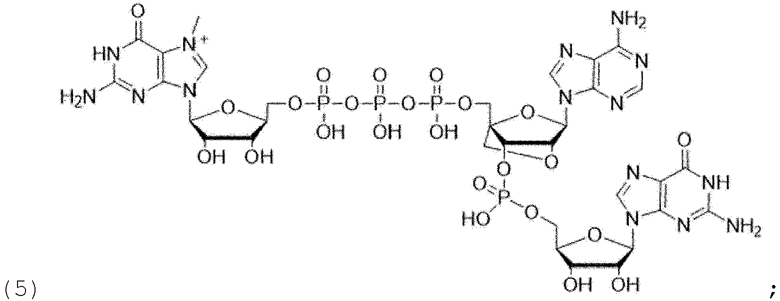 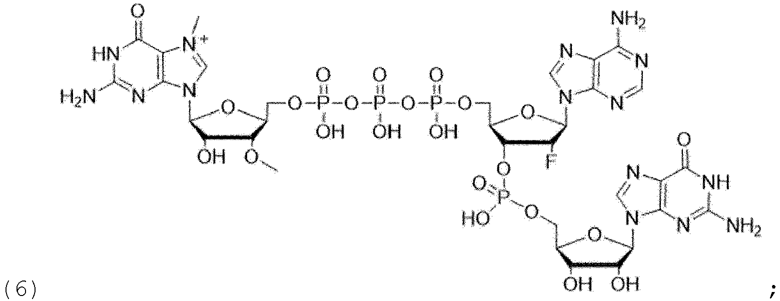 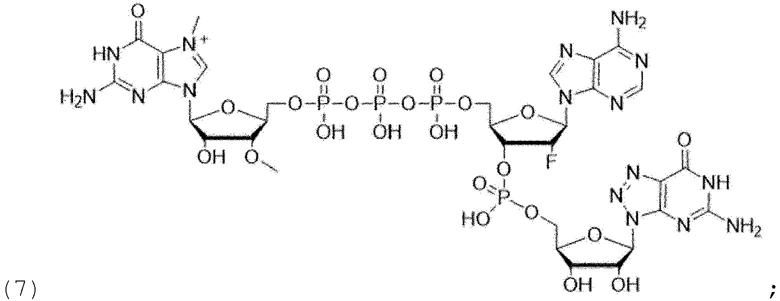 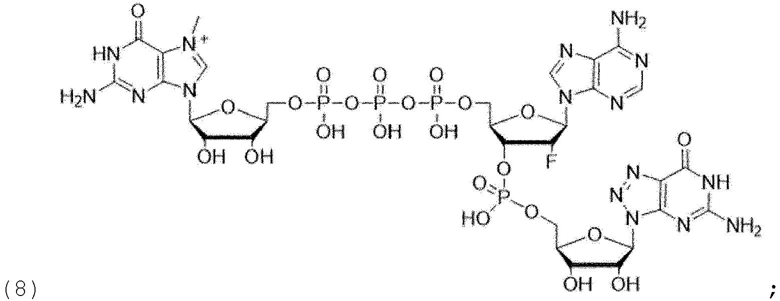 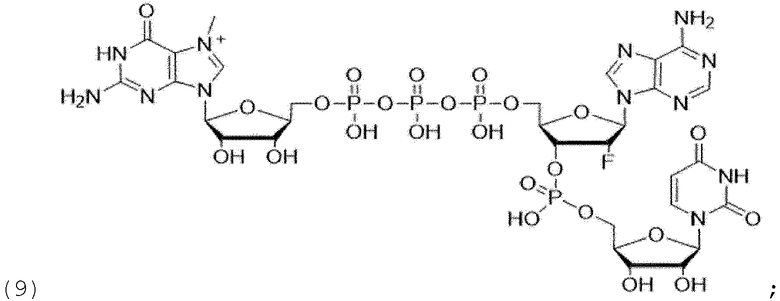 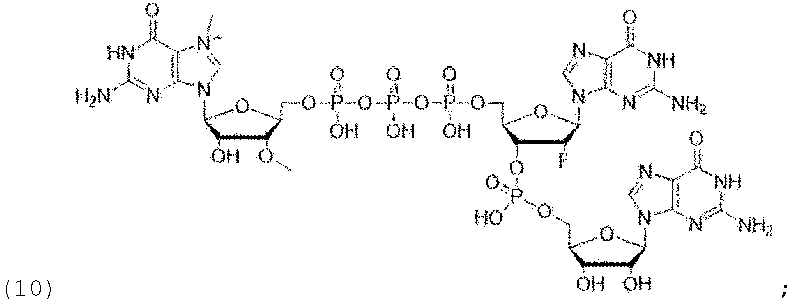 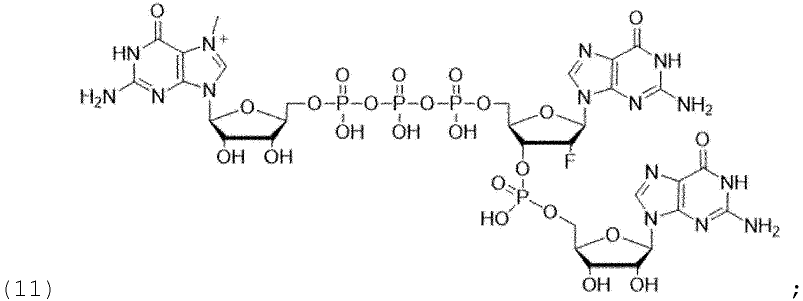 and

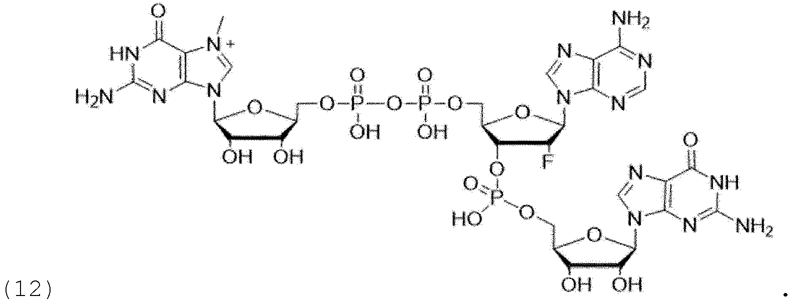

According to an embodiment of the present invention, the compound represented by Formula 1 may be at least one selected from the group consisting of the following compounds:
2-amino-9-((2R,3R,4S,5R)-5-((((((((((2R,3R,4R,5R)-3-(((((2R,3S,4R,5R)-5-(2-amino-6-oxo-1,6-dihydro-9H-purin-9-yl)-3,4-dihydroxytetrahydrofuran-2-yl)methoxy)(hydroxy)phosphoryl)oxy)-5-(6-amino-9H-purin-9-yl)-4-fluorotetrahydrofuran-2-yl)methoxy)(hydroxy)phosphoryl)oxy)(hydroxy)phosphoryl)oxy) (hydroxy)phosphoryl)oxy)methyl)-3,4-dihydroxytetrahydrofuran-2-yl)-7-methyl-6-oxo-6,9-dihydro-1H-purin-7-ium **(oligonucleotide 1);**
2-amino-9-((2R, 3R, 4S, 5R)-5-((((((((((2R, 3R, 4R, 5R)-3-(((((2R,3S,4R,5R)-5-(2-amino-6-oxo-1,6-dihydro-9H-purin-9-yl)-3,4-dihydroxytetrahydrofuran-2-yl)methoxy)(hydroxy)phosphoryl)oxy)-5-(6-amino-9H-purin-9-yl)-4-(2-methoxyethoxy)tetrahydrofuran-2-yl)methoxy)(hydroxy)phosphoryl)oxy)(hydroxy)phosphoryl)oxy) (hydroxy)phosphoryl)oxy)methyl)-3,4-dihydroxytetrahydrofuran-2-yl)-7-methyl-6-oxo-6,9-dihydro-1H-purin-7-ium **(oligonucleotide 2);**
2-amino-9-((2S,3S,4R,5S)-5-((((((((((1S,3R,4R,6S,7S)-7-(((((2R,3S,4R,5R)-5-(2-amino-6-oxo-1,6-dihydro-9H-purin-9-yl)-3,4-dihydroxytetrahydrofuran-2-yl)methoxy)(hydroxy)phosphoryl)oxy)-3-(6-amino-9H-purin-9-yl)-6-methyl-2,5-dioxabicyclo[2.2.1]heptan-1-yl)methoxy)(hydroxy)phosphoryl)oxy)(hydroxy)phosphoryl)oxy) (hydroxy)phosphoryl)oxy)methyl)-3,4-dihydroxytetrahydrofuran-2-yl)-7-methyl-6-oxo-6,9-dihydro-1H-purin-7-ium **(oligonucleotide 3);**
2-amino-9-((2S,3S,4R,5S)-5-((((((((((2R,3R,4R,5R)-3-(((((2R,3S,4R,5R)-5-(5-amino-7-oxo-6,7-dihydro-3H-[1,2,3]triazolo[4,5-d]pyrimidin-3-yl)-3,4-dihydroxytetrahydrofuran-2-yl)methoxy)(hydroxy)phosphoryl)oxy)-5-(6-amino-9H-purin-9-yl)-4-methoxytetrahydrofuran-2-yl)methoxy)(hydroxy)phosphoryl)oxy)(hydroxy)phosphoryl)oxy) hydroxy)phosphoryl)oxy)methyl)-3,4-dihydroxytetrahydrofuran-2-yl)-7-methyl-6-oxo-6,9-dihydro-1H-purin-7-ium **(oligonucleotide 4);**
2-amino-9-((2S,3S,4R,5S)-5-((((((((((1R,3R,4R,7S)-7-(((((2R,3S,4R,5R)-5-(2-amino-6-oxo-1,6-dihydro-9H-purin-9-yl)-3,4-dihydroxytetrahydrofuran-2-yl)methoxy)(hydroxy)phosphoryl)oxy)-3-(6-amino-9H-purin-9-yl)-2,5-dioxabicyclo[2.2.1]heptan-1-yl)methoxy)(hydroxy)phosphoryl)oxy)(hydroxy)phosphoryl)oxy) hydroxy)phosphoryl)oxy)methyl)-3,4-dihydroxytetrahydrofuran-2-yl)-7-methyl-6-oxo-6,9-dihydro-1H-purin-7-ium **(oligonucleotide 5);**
2-amino-9-((2S,3S,4R,5S)-5-((((((((((2R,3R,4R,5R)-3-(((((2R,3S,4R,5R)-5-(2-amino-6-oxo-1,6-dihydro-9H-purin-9-yl)-3,4-dihydroxytetrahydrofuran-2-yl)methoxy)(hydroxy)phosphoryl)oxy)-5-(6-amino-9H-purin-9-yl)-4-fluorotetrahydrofuran-2-yl)methoxy)(hydroxy)phosphoryl)oxy)(hydroxy)phosphoryl)oxy) hydroxy)phosphoryl)oxy)methyl)-3-hydroxy-4-methoxytetrahydrofuran-2-yl)-7-methyl-6-oxo-6,9-dihydro-1H-purin-7-ium **(oligonucleotide 6);**
2-amino-9-((2S,3S,4R,5S)-5-((((((((((2R,3R,4R,5R)-3-(((((2R,3S,4R,5R)-5-(5-amino-7-oxo-6,7-dihydro-3H-[1,2,3]triazolo[4,5-d]pyrimidin-3-yl)-3,4-dihydroxytetrahydrofuran-2-yl)methoxy)(hydroxy)phosphoryl)oxy)-5-(6-amino-9H-purin-9-yl)-4-fluorotetrahydrofuran-2-yl)methoxy)(hydroxy)phosphoryl)oxy)(hydroxy)phosphoryl)oxy) hydroxy)phosphoryl)oxy)methyl)-3-hydroxy-4-methoxytetrahydrofuran-2-yl)-7-methyl-6-oxo-6,9-dihydro-1H-purin-7-ium **(oligonucleotide 7);**
2-amino-9-((2S,3S,4R,5S)-5-((((((((((2R,3R,4R,5R)-3-(((((2R,3S,4R,5R)-5-(5-amino-7-oxo-6,7-dihydro-3H-[1,2,3]triazolo[4,5-d]pyrimidin-3-yl)-3,4-dihydroxytetrahydrofuran-2-yl)methoxy)(hydroxy)phosphoryl)oxy)-5-(6-amino-9H-purin-9-yl)-4-fluorotetrahydrofuran-2-yl)methoxy)(hydroxy)phosphoryl)oxy)(hydroxy)phosphoryl)oxy) hydroxy)phosphoryl)oxy)methyl)-3,4-dihydroxytetrahydrofuran-2-yl)-7-methyl-6-oxo-6,9-dihydro-1H-purin-7-ium **(oligonucleotide 8);**
2-amino-9-((2S,3S,4R,5S)-5-((((((((((2R,3R,4R,5R)-5-(6-amino-9H-purin-9-yl)-3-(((((2R,3S,4R,5R)-5-(2,4-dioxo-3,4-dihydropyrimidin-1(2H)-yl)-3,4-dihydroxytetrahydrofuran-2-yl)methoxy)(hydroxy)phosphoryl)oxy)-4-fluorotetrahydrofuran-2-yl)methoxy)(hydroxy)phosphoryl)oxy)(hydroxy)phosphoryl)oxy) hydroxy)phosphoryl)oxy)methyl)-3,4-dihydroxytetrahydrofuran-2-yl)-7-methyl-6-oxo-6,9-dihydro-1H-purin-7-ium **(oligonucleotide 9);**
2-amino-9-((2S, 3S, 4R, 5S)-5-((((((((((2R, 3R, 4R, 5R)-5-(2-amino-6-oxo-1,6-dihydro-9H-purin-9-yl)-3-(((((2R,3S,4R,5R)-5-(2-amino-6-oxo-1,6-dihydro-9H-purin-9-yl)-3,4-dihydroxytetrahydrofuran-2-yl)methoxy)(hydroxy)phosphoryl)oxy)-4-fluorotetrahydrofuran-2-yl)methoxy)(hydroxy)phosphoryl)oxy)(hydroxy)phosphoryl)oxy) hydroxy)phosphoryl)oxy)methyl)-3-hydroxy-4-methoxytetrahydrofuran-2-yl)-7-methyl-6-oxo-6,9-dihydro-1H-purin-7-ium **(oligonucleotide 10);**
2-amino-9-((2S,3S,4R,5S)-5-((((((((((2R,3R,4R,5R)-5-(2-amino-6-oxo-1,6-dihydro-9H-purin-9-yl)-3-(((((2R,3S,4R,5R)-5-(2-amino-6-oxo-1,6-dihydro-9H-purin-9-yl)-3,4-dihydroxytetrahydrofuran-2-yl)methoxy)(hydroxy)phosphoryl)oxy)-4-fluorotetrahydrofuran-2-yl)methoxy)(hydroxy)phosphoryl)oxy)(hydroxy)phosphoryl)oxy) hydroxy)phosphoryl)oxy)methyl)-3,4-dihydroxytetrahydrofuran-2-yl)-7-methyl-6-oxo-6,9-dihydro-1H-purin-7-ium **(oligonucleotide 11);** and
2-amino-9-((2S,3S,4R,5S)-5-((((((((2R,3R,4R,5R)-3-(((((2R,3S,4R,5R)-5-(2-amino-6-oxo-1,6-dihydro-9H-purin-9-yl)-3,4-dihydroxytetrahydrofuran-2-yl)methoxy)(hydroxy)phosphoryl)oxy)-5-(6-amino-9H-purin-9-yl)-4-fluorotetrahydrofuran-2-yl)methoxy)(hydroxy)phosphoryl)oxy)(hydroxy)phosphoryl)oxy) methyl)-3,4-dihydroxytetrahydrofuran-2-yl)-7-methyl-6-oxo-6,9-dihydro-1H-purin-7-ium **(oligonucleotide 12).**

According to an embodiment of the present invention, the compound represented by Formula 1 may be an oligonucleotide primer for RNA capping.

In the present invention, the term "stereoisomer" means a compound or salt thereof of the present invention having the same Formula or molecular formula but different in spatial arrangement. Each of these stereoisomers and mixtures thereof are also included within the scope of the present invention. In addition, unless otherwise specified, a solid bond (-) connected to an asymmetric carbon atom may include a wedged bond ( 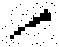 ) or wedge dashed bond ( 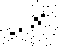 ) representing the absolute arrangement of stereocenters.

In the present invention, the term "salt" means a salt commonly used in the pharmaceutical industry. Specifically, the salt may be a base addition salt. Examples of the salt may include inorganic ion salts prepared with, for example, calcium, potassium, sodium or magnesium, and the like; amino acid salts prepared with arginine, lysine, histidine, and the like; and amine salts prepared with trimethylamine, triethylamine, ammonia, pyridine, picoline, and the like; but the types of salts meant in the present invention are not limited by these listed salts.

### RNA capped with novel oligonucleotide primer

The oligonucleotide primer for RNA capping as described above may be used to produce an RNA molecule comprising the oligonucleotide primer. Accordingly, the present invention provides an RNA molecule comprising an oligonucleotide primer for RNA capping represented by Formula 1.

The oligonucleotide primer for RNA capping according to the present invention may be extended by RNA polymerase through incorporation of NTP onto the 3'-end. In vitro transcription may be initiated under the control of a promoter in a transcription system containing essential components such as a DNA template (for example, DNA plasmid), RNA polymerase, nucleoside 5'-triphosphate, and appropriate buffers. Here, the oligonucleotide primer is complementary to the DNA template at an initiation site.

In the present invention, the term promoter refers to a specific region of dsDNA template that induces and controls the initiation of transcription of a specific DNA sequence (for example, gene). The promoter is located on the same strand and upstream (toward the 5' region of the sense strand) on the DNA. The promoter is typically immediately adjacent to or partially overlaps the DNA sequence to be transcribed. The location of nucleotides in the promoter is designed based on the transcriptional start site where transcription of DNA begins (position +1). The initiation oligonucleotide primer is complementary to the initiation site of the promoter sequence, wherein in certain embodiments, it is at positions +1 and +2, and in the case of initiation tetramers, at positions +1, +2 and +3).

In an embodiment, the oligonucleotide primer for RNA capping is attached to the 5' upstream end of the RNA molecule to form a 5'-capped RNA. RNA molecules thus formed include, but are not limited to, mRNA, small nuclear RNA (snRNA), small nucleolar RNA (snoRNA), and small Cajal bodyspecific RNA (scaRNA).

In an embodiment, the RNA molecule may be mRNA and may contain at least one coding sequence (CDS). Further, the RNA molecule may comprise a polyA sequence and/or a polyadenylation signal. The polyA sequence may be comprised entirely or mostly of adenine nucleotides or analogs or derivatives thereof. The polyA sequence may be a tail located adjacent to a 3' untranslated region of a nucleic acid.

The present invention provides a method for synthesizing a 5'-capped RNA molecule, comprising: (S-1) mixing a DNA template, the oligonucleotide primer for RNA capping represented by Formula 1 as described above, and RNA polymerase; and (S-2) performing transcription of the polynucleotide template by incubating the mixture.

Meanwhile, methods for DNA-templated and promoter controlled synthesis of RNA using oligonucleotide primers, RNA polymerase, and nucleoside 5'-triphosphate (NTP) are known in the technical field to which the present invention belongs. Therefore, it is possible to utilize the oligonucleotide primer for RNA capping represented by Formula 1 provided in the present invention, thereby synthesizing an RNA molecule comprising the same.

According to an embodiment of the present invention, a DNA template may be a double-stranded linear DNA, a partially double-stranded linear DNA, a circular double-stranded DNA, a DNA plasmid, a PCR amplicon, or in addition, may be a modified nucleic acid capable of properly reacting with RNA polymerase.

According to an embodiment of the present invention, the synthesizing of the RNA molecule may be performed in vitro. In order to perform large-scale transcription in vitro, single subunit phage polymerase derived from T7, T3, SP6, K1-5, K1E, K1F or K11 bacteriophage may be used. This family of polymerases requires no accessory proteins and has simple, minimal promoter sequences of about 17 nucleotides which have minimal constraints of the initiating nucleotide sequence. One example of the RNA polymerase capable of being used in the present invention may be T7 RNA polymerase (T7 RNAP), but those skilled in the art will understand that the present invention could also be practiced using other RNA polymerases. Unlike DNA polymerases, T7 RNAP initiates RNA synthesis in the absence of a primer. The first step in initiation is called de novo RNA synthesis, in which RNA polymerase recognizes a specific sequence on the DNA template, selects the first pair of nucleotide triphosphates complementary to template residues at positions +1 and +2, and catalyzes the formation of a phosphodiester bond to form a dinucleotide.

### Medical use of capped RNA

The 5'-capped RNA molecule according to the present invention as described above may be usefully utilized for medical purposes.

According to an embodiment of the present invention, the 5'-capped RNA molecule produced according to the present invention may be utilized as nucleic acid therapeutic agents or vaccines. For example, the nucleic acid therapeutic agent or vaccine may be used as an RNA vaccine (vaccine for preventing cancer or infectious diseases). The nucleic acid therapeutic agent or vaccine may be administered to a subject and translated in vivo to produce the desired peptide.

According to an embodiment of the present invention, the 5'-capped RNA molecule may be introduced into a cell to produce a protein capable of treating a medical condition of the cell or having a therapeutic effect on the cell.

According to an embodiment of the present invention, the nucleic acid therapeutic agent or vaccine may further comprise a carrier capable of introducing the RNA molecule into a target cell together with the RNA molecule.

According to an embodiment of the present invention, the present invention provides a peptide translated from the 5'-capped RNA molecule as described above.

According to an embodiment of the present invention, the present invention provides a cell into which the 5'-capped RNA molecule as described above is introduced. The cell may be a somatic cell of a subject, or may be a cell line capable of being cultured in vitro.

According to an embodiment of the present invention, the present invention provides a peptide produced from the cell into which the 5'-capped RNA molecule is introduced.

In the present invention, pharmaceutical compositions for RNA therapeutic agents or vaccines may be formulated for administration by injection or by other suitable routes known to those skilled in the art to treat a particular condition. An injectable composition for parenteral administration typically contains the active compound in a suitable solution and/or pharmaceutical carrier such as sterile physiological saline. The composition may also be formulated as a suspension in a lipid or phospholipid, a liposomal suspension, or an aqueous emulsion.

Methods of preparing compositions and/or formulations for RNA therapeutic agents or vaccines are known to those skilled in the art [see Remington's Pharmaceutical Sciences (19th Ed., Williams & Wilkins, 1995)]. The composition to be administered will contain a specific amount of the selected compound in a pharmaceutically safe and effective amount for increasing expression of the desired protein in the target cells or tissue.

In some embodiments, the pharmaceutical composition contains at least 0.1% (w/v) of the compound as described above, in some embodiments, the pharmaceutical composition contains greater than 0.1%, in some embodiments, the pharmaceutical composition contains up to about 10%, in some embodiments, the pharmaceutical composition contains up to about 5%, and in some embodiments, the pharmaceutical composition contains up to about 1% (w/v) of the compound. Choice of a suitable concentration depends on factors such as the desired dose, frequency and delivery method of the active agent.

For treatment of a subject, such as a mammal or a human, dosages are determined based on factors such as the weight and overall health of the subject, the condition treated, severity of symptoms, and the like. Dosages and concentrations are determined to produce the desired benefit while avoiding any undesirable side effects. Typical dosages of the subject compounds are in the range of about 0.0005 to 500 mg/day for a human patient, and ranging in some embodiments between about 1-100 mg/day. For example, higher dose regimens may include, for example, 50 to 100, 75 to 100, or 50 to 75 mg/day, and lower dose regimens may include, for example, 1 to 50, 25 to 50, or 1 to 25 mg/day.

### [Advantageous Effects]

The novel oligonucleotide according to the present invention may be utilized for the synthesis of 5'-capped RNA to improve not only a RNA production process (for example, synthesis yield, synthesis scale, purity, and the like) but also the efficacy of nucleic acid therapeutic agents or vaccines utilizing the oligonucleotide (for example, RNA stability and/or protein expression efficiency) and to reduce side effects (for example, immunogenicity). Therefore, the present invention may be usefully utilized in the field of nucleic acid therapeutic agents or vaccines.

### [Description of Drawings]

FIG. 1 shows the electrophoresis results for in vitro transcribed mRNA.
FIG. 2 shows the expression results of GFP mRNA through a fluorescence microscope.

### [Best Mode for carrying out the invention]

Hereinafter, the constitution and effects of the present invention will be described in more detail through Examples and Experimental Examples. These Examples and Experimental Examples are only provided for illustrating the present invention, but the scope of the present invention is not limited by these Examples and Experimental Examples.

### Preparation Example 1: Preparation of ((2R,3R,4R,5R)-3-(((((2R,3S,4R,5R)-5-(2-amino-6-oxo-1,6-dihydro-9H-purin-9-yl)-3,4-dihydroxytetrahydrofuran-2-yl)methoxy)(hydroxy)phosphoryl)oxy)-5-(6-amino-9H-purin-9-yl)-4-fluorotetrahydrofuran-2-yl)methyl dihydrogen phosphate. 2 triethylamine salt

### Step 1. Preparation of (2R,3R,4R,5R)-5-(6-benzamido-9H-purin-9-yl)-4-fluoro-2-(hydroxymethyl)tetrahydrofuran-3-yl (2-cyanoethyl) (((3aR,4R,6R,6aR)-6-(2-isobutyramido-6-oxo-1,6-dihydro-9H-purin-9-yl)-2,2-dimethyltetrahydrofuro[3,4-d][1,3]dioxol-4-yl)methyl)phosphate

N-(9-((3aR,4R,6R,6aR)-6-(hydroxymethyl)-2,2-dimethyltetrahydrofuro[3,4-d] [1,3]dioxol-4-yl)-6-oxo-6,9-dihydro-1H-purin-2-yl)isobutyramide (4 g, 10.17 mmol) was dissolved in acetonitrile. After cooling to 0 to 5 °C, (2R,3R,4R,5R)-5-(6-benzamido-9H-purin-9-yl)-2-((bis(4-methoxyphenyl)(phenyl)methoxy)methyl)-4-fluorotetrahydrofuran-3-yl (2-cyanoethyl) diisopropylphosphoramidite (8.91 g, 10.17 mmol) and 1H-tetrazole (2.85 g, 40.7 mmol) were sequentially added. After 10 minutes, the resulting mixture was slowly warmed to room temperature and stirred for 1 hour. The reaction solution was cooled to 0 to 5 °C, and then I₂ (0.1 M) (7.74 g, 30.5 mmol) in THF/distilled water/pyridine (66 : 33 : 1) (101.6 mL) was added. The reaction solution was slowly warmed to room temperature and stirred for 1 hour. After completion of the reaction, the reaction solution was diluted with DCM, 10% sodium thiosulfate pentahydrate solution was added, and an organic layer and an aqueous layer were separated. The organic layer was dehydrated with Na₂SO₄ and concentrated under reduced pressure.

The concentrated residue was dissolved in DCM (200 mL) . After cooling to 0 to 5 °C, TFA (10.17 mL) was diluted in DCM (817 mL), added slowly, and stirred for 10 minutes. The temperature of the reaction solution was slowly raised to room temperature, and the mixture was stirred for 10 minutes. The reaction solution was cooled to 0 to 5 °C, and then the pH was adjusted between 8 to 9 while slowly adding a saturated NaHCO₃ (aq) solution. The organic layer and the aqueous layer were separated, and the organic layer was dried over Na₂SO₄ and filtered. After concentration under reduced pressure and purified by column chromatography, the desired product (5.62 g) was obtained as a yellow solid.

LC-MS (ESI, m/z) = 882.1 (M+H⁺).

### Step 2. Preparation of (2R,3R,4R,5R)-5-(6-benzamido-9H-purin-9-yl)-2-(((bis(2-cyanoethoxy)phosphoryl)oxy)methyl)-4-fluorotetrahydrofuran-3-yl (2-cyanoethyl) (((3aR,4R,6R,6aR)-6-(2-isobutyramido-6-oxo-1,6-dihydro-9H-purin-9-yl)-2,2-dimethyltetrahydrofuro[3,4-d][1,3]dioxol-4-yl)methyl)phosphate

The compound obtained in step 1 above, i.e., (2R,3R,4R,5R)-5-(6-benzamido-9H-purin-9-yl)-4-fluoro-2-(hydroxymethyl)tetrahydrofuran-3-yl (2-cyanoethyl) (((3aR,4R,6R,6aR)-6-(2-isobutyramido-6-oxo-1,6-dihydro-9H-purin-9-yl)-2,2-dimethyltetrahydrofuro[3,4-d] [1,3]dioxol-4-yl)methyl)phosphate (5.6 g, 6.35 mmol) was dissolved in DCM (21.2 mL). After cooling to 0 to 5 °C, bis(2-cyanoethyl)diisopropylphosphoramidite (3.32 mL, 12.70 mmol) and 1H-tetrazole (0.890 g, 12.70 mmol) were sequentially added thereto. After 10 minutes, the resulting mixture was slowly warmed to room temperature and stirred for 1 hour. The reaction solution was cooled to 0 to 5 °C, and then I₂ (0.1 M) (4.84 g, 19.05 mmol) in THF/distilled water/pyridine (66 : 33 : 1) (63.6 mL) was added. The reaction solution was slowly warmed to room temperature and stirred for 1 hour. After completion of the reaction, the reaction solution was diluted with DCM, 10% sodium thiosulfate pentahydrate solution was added, and an organic layer and an aqueous layer were separated. The organic layer was dehydrated with Na₂SO₄, concentrated under reduced pressure, and purified by column chromatography to afford the desired product (5.78 g) as a yellow solid.

LC-MS (ESI, m/z) = 1086.1 (M+H⁺).

### Step 3. Preparation of ((2R,3R,4R,5R)-3-(((((2R,3S,4R,5R)-5-(2-amino-6-oxo-1,6-dihydro-9H-purin-9-yl)-3,4-dihydroxytetrahydrofuran-2-yl)methoxy)(hydroxy)phosphoryl)oxy)-5-(6-amino-9H-purin-9-yl)-4-fluorotetrahydrofuran-2-yl)methyl dihydrogen phosphate. 2 triethylamine salt

The compound obtained in step 2 above, i.e., (2R,3R,4R,5R)-5-(6-benzamido-9H-purin-9-yl)-2-(((bis(2-cyanoethoxy)phosphoryl)oxy)methyl)-4-fluorotetrahydrofuran-3-yl (2-cyanoethyl) (((3aR,4R,6R,6aR)-6-(2-isobutyramido-6-oxo-1,6-dihydro-9H-purin-9-yl)-2,2-dimethyltetrahydrofuro[3,4-d][1,3]dioxol-4-yl)methyl)phosphate (2.89 g, 2.71 mmol) was dissolved in 1M HCl aqueous solution (162 ml, 162 mmol), and stirred at room temperature for 16 hours. After cooling the reaction solution to 0 to 5 °C, ammonia water (42.2 mL, 1083 mmol) was slowly added. The reaction solution was slowly warmed to room temperature, and heated to 50 to 55 °C for 23 hours. The reaction solution was washed with DCM (2 times with 150 mL), and the aqueous layer was purified with DEAE Sephadex resin to afford the desired product (1.98 g) as a white solid.

LC-MS (ESI, m/z) = 695.1 (M+H⁺).

### Preparation Example 2: Preparation of 2-amino-9-((2R,3R,4S,5R)-3,4-dihydroxy-5-(((hydroxy((hydroxy(1H-imidazol-1-yl)phosphoryl)oxy)phosphoryl)oxy)methyl)tetrahydrofuran-2-yl)-7-methyl-6-oxo-6,9-dihydro-1H-purin-7-ium. sodium salt

### Step 1. Preparation of 2-amino-9-((2R,3R,4S,5R)-3,4-dihydroxy-5-(((hydroxy(phosphonooxy)phosphoryl)oxy)methyl)tetrahydrofur an-2-yl)-7-methyl-6-oxo-6,9-dihydro-1H-purin-7-ium.triethylamine salt

((2R,3S,4R,5R)-5-(2-amino-6-oxo-1,6-dihydro-9H-purin-9-yl)-3,4-dihydroxytetrahydrofuran-2-yl)methyl trihydrogen diphosphate. 2triethylamine salt (5 g, 7.74 mmol) was dissolved in purified water (155 mL) and the pH of the solution was adjusted to 4.5 with acetic acid. After slowly adding dimethyl sulfate (5.18 mL, 54.2 mmol) for 1 minute, the resulting solution was stirred at room temperature for 1 hour. Here, the pH of the solution was adjusted to 4 to 4.5 using 5N NaOH aqueous solution. After completion of the reaction, the mixture was washed with DCM (200 mL × 3 times), and the aqueous layer was adjusted to pH 5.5 to 6 with 1M TEAB. The reaction product was purified with DEAE Sephadex resin to afford the desired product (3.03 g) as a white solid.

LC-MS (ESI, m/z) = 456.0 (M-H⁺).

### Step 2. Preparation of 2-amino-9-((2R,3R,4S,5R)-3,4-dihydroxy-5-(((hydroxy((hydroxy(1H-imidazol-1-yl)phosphoryl)oxy)phosphoryl)oxy)methyl)tetrahydrofuran-2-yl)-7-methyl-6-oxo-6,9-dihydro-1H-purin-7-ium. sodium salt

2-Amino-9-((2R,3R,4S,5R)-3,4-dihydroxy-5-(((hydroxy(phosphonooxy)phosphoryl)oxy)methyl)tetrahydrofur an-2-yl)-7-methyl-6-oxo-6,9-dihydro-1H-purin-7-ium. triethylamine salt (3.03 g, 5.42 mmol) obtained in step 1 above and imidazole (3.69 g, 54.2 mmol) were dissolved in DMF (38.7 mL). After adding 2,2'-dithiodipyridine (5.97 g, 27.1 mmol), the mixture was stirred at room temperature for 10 hours. After completion of the reaction, the reaction solution was cooled to -10 to -20 °C, and 0.1M sodium percholate in acetone (108 mL, 10.83 mmol) was added dropwise. The resulting solid was filtered and dried to afford the desired product (2.33 g) as a white solid.

LC-MS (ESI, m/z) = 507.0 (M-H⁺).

### Preparation Example 3: Preparation of 2-amino-9-((2R,3R,4S,5R)-3-dihydroxy-5-(((hydroxy((hydroxy(1H-imidazol-1-yl)phosphoryl)oxy)phosphoryl)oxy)methyl)-4-methoxytetrahydrofuran-2-yl)-7-methyl-6-oxo-6,9-dihydro-1H-purin-7-ium. sodium salt

2-Amino-9-((2R,3R,4S,5R)-3,4-dihydroxy-5-(((hydroxy(phosphonooxy)phosphoryl)oxy)methyl)tetrahydrofur an-2-yl)-7-methyl-6-oxo-6,9-dihydro-1H-purin-7-ium. 2 triethylamine salt (190 mg, 0.331 mmol) was used to react in a similar manner to that of Preparation Example 2 to afford the desired product (159 mg) as a white solid.

LC-MS (ESI, m/z) = 522.0 (M+H⁺).

### Example 1: Preparation of 2-amino-9-((2R,3R,4S,5R)-5-((((((((((2R,3R,4R,5R)-3-(((((2R,3S,4R,5R)-5-(2-amino-6-oxo-1,6-dihydro-9H-purin-9-yl)-3,4-dihydroxytetrahydrofuran-2-yl)methoxy)(hydroxy)phosphoryl)oxy)-5-(6-amino-9H-purin-9-yl)-4-fluorotetrahydrofuran-2-yl)methoxy)(hydroxy)phosphoryl)oxy)(hydroxy)phosphoryl)oxy) (hydroxy)phosphoryl)oxy)methyl)-3,4-dihydroxytetrahydrofuran-2-yl)-7-methyl-6-oxo-6,9-dihydro-1H-purin-7-ium.3 triethylamine salt (oligonucleotide 1)

The compound (20 mg, 0.029 mmol) obtained in Preparation Example 1 and the compound (23.18 mg, 0.041 mmol) obtained in Preparation Example 2 were dissolved in DMSO (0.29 mL), and MgCl₂ (3.84 mg, 0.041 mmol) was added thereto, followed by stirring at room temperature for 7 hours. After the reaction was completed, the reaction mixture was diluted in 1.44 mL of 0.25 mM EDTA 2Na salt solution (aq). After confirming complete dissolution, the reaction mixture was diluted in 144 mL of purified water and purified with DEAE Sephadex resin to afford the desired product (22.4 mg) as a white solid.

LC-MS (ESI, m/z) = 1132.6 (M-H⁺).

### Example 2: Preparation of 2-amino-9-((2R,3R,4S,5R)-5-((((((((((2R,3R,4R,5R)-3-(((((2R,3S,4R,5R)-5-(2-amino-6-oxo-1,6-dihydro-9H-purin-9-yl)-3,4-dihydroxytetrahydrofuran-2-yl)methoxy)(hydroxy)phosphoryl)oxy)-5-(6-amino-9H-purin-9-yl)-4-(2-methoxyethoxy)tetrahydrofuran-2-yl)methoxy)(hydroxy)phosphoryl)oxy)(hydroxy)phosphoryl)oxy) (hydroxy)phosphoryl)oxy)methyl)-3,4-dihydroxytetrahydrofuran-2-yl)-7-methyl-6-oxo-6,9-dihydro-1H-purin-7-ium.3 triethylamine salt (oligonucleotide 2)

((2R,3R,4R,5R)-3-(((((2R,3S,4R,5R)-5-(2-amino-6-oxo-1,6-dihydro-9H-purin-9-yl)-3,4-dihydroxytetrahydrofuran-2-yl)methoxy)(hydroxy)phosphoryl)oxy)-5-(6-amino-9H-purin-9-yl)-4-(2-methoxyethoxy)tetrahydrofuran-2-yl)methyl dihydrogen phosphate. 2-triethylamine salt (92 mg, 0.123 mmol) obtained by synthesis in a similar manner to that of Preparation Example 1 and the compound (91.3 mg, 0.172 mmol) obtained in Preparation Example 2 were reacted in the same manner as in Example 1 to afford the desired product (73 mg) as a white solid.

LC-MS (ESI, m/z) = 1187.7 (M-3H⁺)

### Example 3: Preparation of 2-amino-9-((2S,3S,4R,5S)-5-((((((((((1S,3R,4R,6S,7S)-7-(((((2R,3S,4R,5R)-5-(2-amino-6-oxo-1,6-dihydro-9H-purin-9-yl)-3,4-dihydroxytetrahydrofuran-2-yl)methoxy)(hydroxy)phosphoryl)oxy)-3-(6-amino-9H-purin-9-yl)-6-methyl-2,5-dioxabicyclo[2.2.1]heptan-1-yl)methoxy)(hydroxy)phosphoryl)oxy)(hydroxy)phosphoryl)oxy) (hydroxy)phosphoryl)oxy)methyl)-3,4-dihydroxytetrahydrofuran-2-yl)-7-methyl-6-oxo-6,9-dihydro-1H-purin-7-ium. 3 triethylamine salt (oligonucleotide 3)

((1S,3R,4R,6S,7S)-7-(((((2R,3S,4R,5R)-5-(2-amino-6-oxo-1,6-dihydro-9H-purin-9-yl)-3,4-dihydroxytetrahydrofuran-2-yl)methoxy)(hydroxy)phosphoryl)oxy)-3-(6-amino-9H-purin-9-yl)-6-methyl-2,5-dioxabicyclo[2.2.1]heptan-1-yl)methyl dihydrogen phosphate. 2-triethylamine salt (30 mg, 0.034 mmol) obtained by synthesis in a similar manner to that of Preparation Example 1 and the compound (35 mg, 0.069 mmol) obtained in Preparation Example 2 were reacted in the same manner as in Example 1 to afford the desired product (6 mg) as a white solid.

LC-MS (ESI, m/z) = 1156.1 (M-H⁺)

### Example 4: Preparation of 2-amino-9-((2S,3S,4R,5S)-5-((((((((((2R,3R,4R,5R)-3-(((((2R,3S,4R,5R)-5-(5-amino-7-oxo-6,7-dihydro-3H-[1,2,3]triazolo[4,5-d]pyrimidin-3-yl)-3,4-dihydroxytetrahydrofuran-2-yl)methoxy)(hydroxy)phosphoryl)oxy)-5-(6-amino-9H-purin-9-yl)-4-methoxytetrahydrofuran-2-yl)methoxy)(hydroxy)phosphoryl)oxy)(hydroxy)phosphoryl)oxy) hydroxy)phosphoryl)oxy)methyl)-3,4-dihydroxytetrahydrofuran-2-yl)-7-methyl-6-oxo-6,9-dihydro-1H-purin-7-ium. 3 triethylamine salt (oligonucleotide 4)

((2R,3R,4R,5R)-3-(((((2R,3S,4R,5R)-5-(5-amino-7-oxo-6,7-dihydro-3H-[1,2,3]triazolo[4,5-d]pyrimidin-3-yl)-3,4-dihydroxytetrahydrofuran-2-yl)methoxy)(hydroxy)phosphoryl)oxy)-5-(6-amino-9H-purin-9-yl)-4-methoxytetrahydrofuran-2-yl)methyl dihydrogen phosphate. 2 triethylamine salt (17 mg, 0.024 mmol) obtained by synthesis in a similar manner to that of Preparation Example 1 and the compound (24.43 mg, 0.048 mmol) obtained in Preparation Example 2 were reacted in the same manner as in Example 1 to afford the desired product (3.8 mg) as a white solid.

LC-MS (ESI, m/z) = 1145.8 (M-2H⁺)

### Example 5: Preparation of 2-amino-9-((2S,3S,4R,5S)-5-((((((((((1R,3R,4R,7S)-7-(((((2R,3S,4R,5R)-5-(2-amino-6-oxo-1,6-dihydro-9H-purin-9-yl)-3,4-dihydroxytetrahydrofuran-2-yl)methoxy)(hydroxy)phosphoryl)oxy)-3-(6-amino-9H-purin-9-yl)-2,5-dioxabicyclo[2.2.1]heptan-1-yl)methoxy)(hydroxy)phosphoryl)oxy)(hydroxy)phosphoryl)oxy) hydroxy)phosphoryl)oxy)methyl)-3,4-dihydroxytetrahydrofuran-2-yl)-7-methyl-6-oxo-6,9-dihydro-1H-purin-7-ium. 3 triethylamine salt (oligonucleotide 5)

((1R,3R,4R,7S)-7-(((((2R,3S,4R,5R)-5-(2-amino-6-oxo-1,6-dihydro-9H-purin-9-yl)-3,4-dihydroxytetrahydrofuran-2-yl)methoxy)(hydroxy)phosphoryl)oxy)-3-(6-amino-9H-purin-9-yl)-2,5-dioxabicyclo[2.2.1]heptan-1-yl)methyl dihydrogen phosphate. 2 triethylamine salt (50 mg, 0.055 mmol) obtained by synthesis in a similar manner to that of Preparation Example 1 and the compound (112.0 mg, 0.221 mmol) obtained in Preparation Example 2 were reacted in the same manner as in Example 1 to afford the desired product (8 mg) as a white solid.

LC-MS (ESI, m/z) = 1143.0 (M-H⁺)

### Example 6: Preparation of 2-amino-9-((2S,3S,4R,5S)-5-((((((((((2R,3R,4R,5R)-3-(((((2R,3S,4R,5R)-5-(2-amino-6-oxo-1,6-dihydro-9H-purin-9-yl)-3,4-dihydroxytetrahydrofuran-2-yl)methoxy)(hydroxy)phosphoryl)oxy)-5-(6-amino-9H-purin-9-yl)-4-fluorotetrahydrofuran-2-yl)methoxy)(hydroxy)phosphoryl)oxy)(hydroxy)phosphoryl)oxy) hydroxy)phosphoryl)oxy)methyl)-3-hydroxy-4-methoxytetrahydrofuran-2-yl)-7-methyl-6-oxo-6,9-dihydro-1H-purin-7-ium. 3 triethylamine salt (oligonucleotide 6)

The compound (30 mg, 0.03 mmol) obtained in Preparation Example 1 and the compound (32.7 mg, 0.06 mmol) obtained in Preparation Example 3 were reacted in the same manner as in Example 1 to afford the desired product (9 mg) as a white solid.

LC-MS (ESI, m/z) = 1149.1 (M+H⁺)

### Example 7: Preparation of 2-amino-9-((2S,3S,4R,5S)-5-((((((((((2R,3R,4R,5R)-3-(((((2R,3S,4R,5R)-5-(5-amino-7-oxo-6,7-dihydro-3H-[1,2,3]triazolo[4,5-d]pyrimidin-3-yl)-3,4-dihydroxytetrahydrofuran-2-yl)methoxy)(hydroxy)phosphoryl)oxy)-5-(6-amino-9H-purin-9-yl)-4-fluorotetrahydrofuran-2-yl)methoxy)(hydroxy)phosphoryl)oxy)(hydroxy)phosphoryl)oxy) hydroxy)phosphoryl)oxy)methyl)-3-hydroxy-4-methoxytetrahydrofuran-2-yl)-7-methyl-6-oxo-6,9-dihydro-1H-purin-7-ium. 3 triethylamine salt (oligonucleotide 7)

((2R,3R,4R,5R)-3-(((((2R,3S,4R,5R)-5-(5-amino-7-oxo-6,7-dihydro-3H-[1,2,3]triazolo[4,5-d]pyrimidin-3-yl)-3,4-dihydroxytetrahydrofuran-2-yl)methoxy)(hydroxy)phosphoryl)oxy)-5-(6-amino-9H-purin-9-yl)-4-fluorotetrahydrofuran-2-yl)methyl dihydrogen phosphate. 2 triethylamine salt (20 mg, 0.029 mmol) obtained by synthesis in a similar manner to that of Preparation Example 1 and the compound (31.3 mg, 0.058 mmol) obtained in Preparation Example 3 were reacted in the same manner as in Example 1 to afford the desired product (16 mg) as a white solid.

LC-MS (ESI, m/z) = 1147.1 (M-2H⁺)

### Example 8: Preparation of 2-amino-9-((2S,3S,4R,5S)-5-((((((((((2R,3R,4R,5R)-3-(((((2R,3S,4R,5R)-5-(5-amino-7-oxo-6,7-dihydro-3H-[1,2,3]triazolo[4,5-d]pyrimidin-3-yl)-3,4-dihydroxytetrahydrofuran-2-yl)methoxy)(hydroxy)phosphoryl)oxy)-5-(6-amino-9H-purin-9-yl)-4-fluorotetrahydrofuran-2-yl)methoxy)(hydroxy)phosphoryl)oxy)(hydroxy)phosphoryl)oxy) hydroxy)phosphoryl)oxy)methyl)-3,4-dihydroxytetrahydrofuran-2-yl)-7-methyl-6-oxo-6,9-dihydro-1H-purin-7-ium. 3 triethylamine salt (oligonucleotide 8)

((2R,3R,4R,5R)-3-(((((2R,3S,4R,5R)-5-(5-amino-7-oxo-6,7-dihydro-3H-[1,2,3]triazolo[4,5-d]pyrimidin-3-yl)-3,4-dihydroxytetrahydrofuran-2-yl)methoxy)(hydroxy)phosphoryl)oxy)-5-(6-amino-9H-purin-9-yl)-4-fluorotetrahydrofuran-2-yl)methyl dihydrogen phosphate. 2 triethylamine salt (20 mg, 0.029 mmol) obtained by synthesis in a similar manner to that of Preparation Example 1 and the compound (30.5 mg, 0.058 mmol) obtained in Preparation Example 2 were reacted in the same manner as in Example 1 to afford the desired product (14.5 mg) as a white solid.

LC-MS (ESI, m/z) = 1133.1 (M-2H⁺)

### Example 9: Preparation of 2-amino-9-((2S,3S,4R,5S)-5-((((((((((2R,3R,4R,5R)-5-(6-amino-9H-purin-9-yl)-3-(((((2R,3S,4R,5R)-5-(2,4-dioxo-3,4-dihydropyrimidin-1(2H)-yl)-3,4-dihydroxytetrahydrofuran-2-yl)methoxy)(hydroxy)phosphoryl)oxy)-4-fluorotetrahydrofuran-2-yl)methoxy)(hydroxy)phosphoryl)oxy)(hydroxy)phosphoryl)oxy) hydroxy)phosphoryl)oxy)methyl)-3,4-dihydroxytetrahydrofuran-2-yl)-7-methyl-6-oxo-6,9-dihydro-1H-purin-7-ium. 3 triethylamine salt (oligonucleotide 9)

((2R,3R,4R,5R)-5-(6-amino-9H-purin-9-yl)-3-(((((2R,3S,4R,5R)-5-(2,4-dioxo-3,4-dihydropyrimidin-1(2H)-yl)-3,4-dihydroxytetrahydrofuran-2-yl)methoxy)(hydroxy)phosphoryl)oxy)-4-fluorotetrahydrofuran-2-yl)methyl dihydrogen phosphate. 2 triethylamine salt (50 mg, 0.058 mmol) obtained by synthesis in a similar manner to that of Preparation Example 1 and the compound (61.8 mg, 0.117 mmol) obtained in Preparation Example 2 were reacted in the same manner as in Example 1 to afford the desired product (34.6 mg) as a white solid.

LC-MS (ESI, m/z) = 1092.8 (M-2H⁺)

### Example 10: Preparation of 2-amino-9-((2S,3S,4R,5S)-5-((((((((((2R,3R,4R,5R)-5-(2-amino-6-oxo-1,6-dihydro-9H-purin-9-yl)-3-(((((2R,3S,4R,5R)-5-(2-amino-6-oxo-1,6-dihydro-9H-purin-9-yl)-3,4-dihydroxytetrahydrofuran-2-yl)methoxy)(hydroxy)phosphoryl)oxy)-4-fluorotetrahydrofuran-2-yl)methoxy)(hydroxy)phosphoryl)oxy)(hydroxy)phosphoryl)oxy) hydroxy)phosphoryl)oxy)methyl)-3-hydroxy-4-methoxytetrahydrofuran-2-yl)-7-methyl-6-oxo-6,9-dihydro-1H-purin-7-ium.3 triethylamine salt (oligonucleotide 10)

((2R,3R,4R,5R)-5-(2-amino-6-oxo-1,6-dihydro-9H-purin-9-yl)-3-((((((2R,3S,4R,5R)-5-(2-amino-6-oxo-1,6-dihydro-9H-purin-9-yl)-3,4-dihydroxytetrahydrofuran-2-yl)methoxy)(hydroxy)phosphoryl)oxy)-4-fluorotetrahydrofuran-2-yl)methyl dihydrogen phosphate. 2 triethylamine salt (20 mg, 0.02 mmol) obtained by synthesis in a similar manner to that of Preparation Example 1 and the compound (21.47 mg, 0.039 mmol) obtained in Preparation Example 3 were reacted in the same manner as in Example 1 to afford the desired product (15.2 mg) as a white solid.

LC-MS (ESI, m/z) = 1165.0 (M+H⁺)

### Example 11: Preparation of 2-amino-9-((2S,3S,4R,5S)-5-((((((((((2R,3R,4R,5R)-5-(2-amino-6-oxo-1,6-dihydro-9H-purin-9-yl)-3-(((((2R,3S,4R,5R)-5-(2-amino-6-oxo-1,6-dihydro-9H-purin-9-yl)-3,4-dihydroxytetrahydrofuran-2-yl)methoxy)(hydroxy)phosphoryl)oxy)-4-fluorotetrahydrofuran-2-yl)methoxy)(hydroxy)phosphoryl)oxy)(hydroxy)phosphoryl)oxy) hydroxy)phosphoryl)oxy)methyl)-3,4-dihydroxytetrahydrofuran-2-yl)-7-methyl-6-oxo-6,9-dihydro-1H-purin-7-ium. 3 triethylamine salt (oligonucleotide 11)

((2R,3R,4R,5R)-5-(2-amino-6-oxo-1,6-dihydro-9H-purin-9-yl)-3-((((((2R,3S,4R,5R)-5-(2-amino-6-oxo-1,6-dihydro-9H-purin-9-yl)-3,4-dihydroxytetrahydrofuran-2-yl)methoxy)(hydroxy)phosphoryl)oxy)-4-fluorotetrahydrofuran-2-yl)methyl dihydrogen phosphate. 2 triethylamine salt (50 mg, 0.049 mmol) obtained by synthesis in a similar manner to that of Preparation Example 1 and the compound (52.3 mg, 0.099 mmol) obtained in Preparation Example 2 were reacted in the same manner as in Example 1 to afford the desired product (26.3 mg) as a white solid.

LC-MS (ESI, m/z) = 1147.9 (M-2H⁺)

### Example 12: Preparation of 2-amino-9-((2S,3S,4R,5S)-5-((((((((2R,3R,4R,5R)-3-(((((2R,3S,4R,5R)-5-(2-amino-6-oxo-1,6-dihydro-9H-purin-9-yl)-3,4-dihydroxytetrahydrofuran-2-yl)methoxy)(hydroxy)phosphoryl)oxy)-5-(6-amino-9H-purin-9-yl)-4-fluorotetrahydrofuran-2-yl)methoxy)(hydroxy)phosphoryl)oxy)(hydroxy)phosphoryl)oxy) methyl)-3,4-dihydroxytetrahydrofuran-2-yl)-7-methyl-6-oxo-6,9-dihydro-1H-purin-7-ium. 3 triethylamine salt (oligonucleotide 12)

The compound (40 mg, 0.047 mmol) obtained in Preparation Example 1 and 2-9-((2S,3S,4R,5S)-3,4-dihydroxy-5-(((hydroxy(1H-imidazol-1-yl)phosphoryl)oxy)methyl)tetrahydrofuran-2-yl)-7-methyl-6-oxo-6,9-dihydro-1H-pyrin-7-ium. sodium salt (42.6 mg, 0.095 mmol) obtained by a method similar to that of Preparation Example 2 were reacted in the same manner as in Example 1 to afford the desired product (6.5 mg) as a white solid.

LC-MS (ESI, m/z) = 1052.1 (M-2H⁺)

### Experimental Example 1: Preparation of GFP DNA template

In order to obtain a DNA template for mRNA synthesis, a synthesis of a plasmid gene having a T7 RNA promoter, 5'UTR, EGFP, 3'UTR, and poly A sequences was performed by Bionics Co., Ltd. Then, in order to change the promoter sequence depending on the cap material, PCR (polymerase chain reaction) was performed by mixing the plasmid gene with primeSTAR HS premix (Takara Bio Inc., catalog # R040A), 2 µmol forward primer (SEQ ID NO: 1), and 2 µmol reverse primer (SEQ ID NO: 2), followed by cloning of the existing plasmid gene with XbaI (Takara Bio Inc., catalog # 1093A) and BamHI (Takara Bio Inc., catalog # 1010A) restriction enzymes to construct a plasmid gene. The constructed plasmid gene was subjected to linearization with SamI (Takara Bio Inc., catalog # 1085A) restriction enzyme to obtain a GFP DNA transcription template (SEQ ID NO: 3) using AccuPrep PCR purification kit (Bioneer Inc., catalog # K-3037).

### Experimental Example 2: In vitro transcription of GFP mRNA

### 2-1: In vitro transcription of non-capped GFP mRNA

For the synthesis reaction of mRNA that does not have a cap structure on the 5' end, 40 mM Tris HCl (pH 7.9), 25 mM magnesium chloride, 2 mM spermidine, 2 mM ATP, 2 mM UTP, 2 mM CTP, 2 mM GTP, 5% DMSO (Sigma Aldrich catalog # 472301-500ML), 5 mg/mL GFP DNA transcription template, 800 U/mL recombinant RNase inhibitory protein (Takara Bio Inc., catalog # 2316A), 2 U/mL yeast inorganic pyrophosphatase (Thermo Fisher Scientific Inc., catalog # EF0221), and 2500 U/mL T7 RNA polymerase (Dyne bio Inc., catalog # dy1670) were mixed.

The transcription reaction mixture was incubated at 37 °C for 5 hours. To the reaction, 40 mM Tris•HCl (pH 7.5), 8 mM magnesium chloride, 5 mM DTT, and 5000 U/mL recombinant DNase I (Takara Bio Inc., catalog # 2270A) were added and incubated at 37 °C for 1 hour. The resulting mRNA was purified using the Zymo Research RNA clean & concentrator-25 kit (catalogue # 1017) according to the manufacturer's instructions.

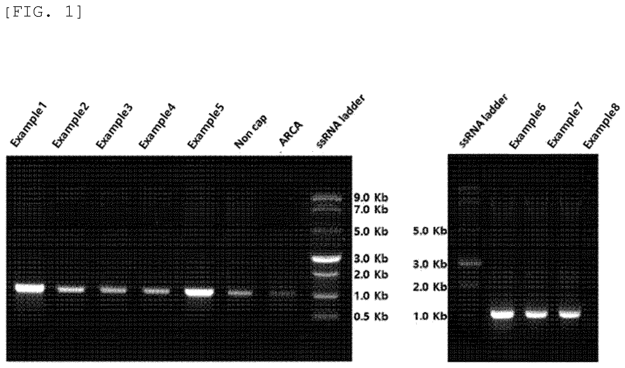

The synthesis of mRNA was confirmed by electrophoresis using a 1% agarose gel added with Lonza GelStar^{™} Nucleic Acid gel Stain (catalogue #5 0535) (FIG. 1). UV analysis of the synthesized mRNA was conducted with a Thermo Scientific^{™} Nanodrop^{™} one UV-Vis spectrometer. As a result, it was confirmed that GFP mRNA containing poly A (120) of about 1066 nt was produced.

### 2-2: In vitro transcription of GFP mRNA via cotranscriptional capping of ARCA cap analogue

For the synthesis reaction of mRNA using ARCA cap, 40 mM Tris HCl (pH 7.9), 25 mM magnesium chloride, 2 mM spermidine, 2 mM ATP, 2 mM UTP, 2 mM CTP, 0.4 mM GTP, 1.6 mM ARCA (TriLink BioTechnologies, catalog # N-7003), 5% DMSO (Sigma Aldrich catalog # 472301-500ML), 5 mg/mL GFP DNA transcription template, 800 U/mL recombinant RNase inhibitory protein (Takara Bio Inc., catalog # 2316A), 2 U/mL yeast inorganic pyrophosphatase (Thermo Fisher Scientific Inc., catalog # EF0221), and 2500 U/mL T7 RNA polymerase (Dyne bio Inc., catalog # dy1670) were mixed.

The transcription reaction mixture was incubated at 37 °C for 5 hours. To the reaction, 40 mM Tris•HCl (pH 7.5), 8 mM magnesium chloride, 5 mM DTT, and 5000 U/mL recombinant DNase I (Takara Bio Inc., catalog # 2270A) were added and incubated at 37 °C for 1 hour. The resulting mRNA was purified using the Zymo Research RNA clean & concentrator-25 kit (catalogue # 1017) according to the manufacturer's instructions.

The synthesis of mRNA was confirmed by electrophoresis using a 1% agarose gel added with Lonza GelStar^{™} Nucleic Acid gel Stain (catalogue #5 0535) (FIG. 1). UV analysis of the synthesized mRNA was measured using a Thermo Scientific^{™} Nanodrop^{™} one UV-Vis spectrometer. As a result, it was confirmed that a dimeric ARCA capping GFP mRNA containing poly A (120) of about 1067 nt was produced.

### 2-3: In vitro transcription of GFP mRNA via cotranscriptional capping of Example

For the synthesis reaction of mRNA using each Example compound of the present invention, 40 mM Tris HCl (pH 7.9), 25 mM magnesium chloride, 2 mM spermidine, 2 mM ATP, 2 mM UTP, 2 mM CTP, 2 mM GTP, 3.2 mM Example compound, 5% DMSO (Sigma Aldrich catalog # 472301-500ML), 5 mg/mL GFP DNA transcription template, 800 U/mL recombinant RNase inhibitory protein (Takara Bio Inc., catalog # 2316A), 2 U/mL yeast inorganic pyrophosphatase (Thermo Fisher Scientific Inc., catalog # EF0221), and 2500 U/mL T7 RNA polymerase (Dyne bio Inc., catalog # dy1670) were mixed.

The transcription reaction mixture was incubated at 37 °C for 5 hours. To the reaction, 40 mM Tris•HCl (pH 7.5), 8 mM magnesium chloride, 5 mM DTT, and 5000 U/mL recombinant DNase I (Takara Bio Inc., catalog # 2270A) were added and incubated at 37 °C for 1 hour. The resulting mRNA was purified using the Zymo Research RNA clean & concentrator-25 kit (catalogue # 1017) according to the manufacturer's instructions.

The synthesis of mRNA was confirmed by electrophoresis using a 1% agarose gel added with Lonza GelStar^{™} Nucleic Acid gel Stain (catalogue #5 0535) (FIG. 1). UV analysis of the synthesized mRNA was measured using a Thermo Scientific^{™} Nanodrop^{™} one UV-Vis spectrometer. As a result, it was confirmed that the capping GFP mRNAs containing poly A (120) of about 1067 nt according to Examples were produced.

### Experimental Example 3: Translation of mRNA in Hela cell

The translational activity of mRNA produced by in vitro transcription was evaluated in a human cervix cancer cell line (Hela). Hela cells were cultured in DMEM supplemented with 10% FBS and 1% penicillin/streptomycin at 37 °C in an atmosphere of 5% CO₂. 1×10⁶ cells/well of Hela cells were plated in a 6-well plate. The following day, cells were transfected using transfection reagent (messengerMAX lipofectamine; Invitrogen, catalog # LMRNA003): According to the transfection reagent manufacturer's instructions, tube A was prepared by diluting 7.5 µL of transfection reagent in 125 µL of complex medium (Opti-MEM; Life technologies) and incubating for 10 minutes at room temperature, and tube B was prepared by diluting 5 µg of prepared mRNA in 125 µL of Opti-MEM. The solutions in tube A and tube B were mixed and incubated for 5 minutes at room temperature. During the incubation time, the cell culture medium was replaced with 10% FBS-containing DMEM without penicillin/streptomycin. Cells were then transfected using the incubated mixed solution. After incubation at 37 °C for 3 to 4 hours in a 5% CO₂ atmosphere, the cell culture medium was replaced with DMEM supplemented with 10% FBS and 1% penicillin/streptomycin. At 96 hours after transfection, the expression of the fluorescent protein was confirmed using a fluorescence microscope.

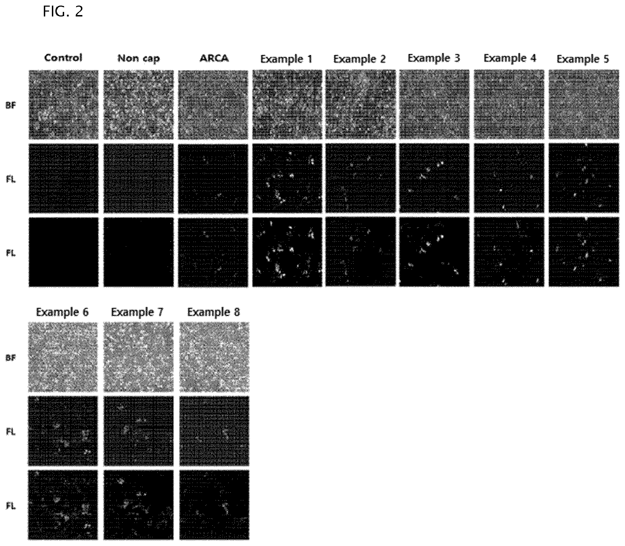

As a result, the mRNAs produced using the Example compounds showed excellent fluorescence activity. On the other hand, mRNA without cap, which is a control group, showed no fluorescence activity at all. In addition, when compared with mRNA synthesized using the ARCA cap, all mRNAs produced using the Example compounds showed excellent protein expression rates (FIG. 2).

### Experimental Example 4: Western blot analysis

After the transfection experiment, the culture medium was removed and washed once using PBS. After treatment with 200 µL of cell lysis buffer (RIPA + phosphatase inhibitor + PMSF), the cells were lysed at 4 °C for 10 minutes. After recovering the cell lysate, centrifugation was performed at 12000 rpm, 4 °C for 10 minutes. Next, the supernatant was recovered, mixed with 5x SDS-sample loading buffer, and then boiled at 100 °C for 5 to 10 minutes. Electrophoresis was performed by running the 10% SDS-PAGE gel at 120V for 10 minutes, and 170V for 1 hour. The sample was transferred at 30V for 1 hour using the Xcell 2 blot Module (Invitrogen). Further, the resulting product was blocked for 1 hour at room temperature using 5% BSA-PBST. The product was reacted overnight at 4 °C with rabbit anti-GFP antibody diluted to 1:1000 in 5% BSA-PBST. Then, the resulting product was washed 3 times for 10 minutes with PBST. The HRP-conjugated rabbit secondary antibody was diluted 1:10000 in 2.5% BSA-PBST and treated for 1 hour at room temperature. Then, the resulting product was washed 3 times for 10 minutes with PBST. After ECL treatment, the obtained product was confirmed by chemiluminescence and the GFP expression level thereof compared to β-actin was analyzed (Table 1).

**[Table 1]**

| | Control | Non cap | ARCA | Example 1 | Example 6 | Example 7 |
|---|---|---|---|---|---|---|
| Relative value | 1.0 | 1.11 | 4.09 | 8.67 | 8.30 | 4.51 |

### Experimental Example 5: Fluorescence-activated cell sorting (FACS) analysis

After the transfection experiment, the culture medium was removed and the residual product was washed once with PBS. After trypsin-EDTA treatment, the sample was incubated at 37 °C for about 2 to 3 minutes until cells began to fall off. The sample was subjected to neutralization with a culture medium (HG DMEM + 10% FBS + 1% penicillin/streptomycin), followed by centrifugation at 1200 rpm for 2 minutes at room temperature. The supernatant was removed, 1 mL of PBS was dispensed, and the clumped cells were released by pipetting. GFP signal (FITC) was measured by FACS (BD FACSDiva 8.0.3) [confirmed by forward scatter (FSC) and side scatter (SSC), shown in Table 2).

**[Table 2]**

| **Materials** | **% parent-P3** | **Relative** |
|---|---|---|
| Negative | 0.4 | 1.0 |
| Non-capped | 4.9 | 12.3 |
| Example 1 | 35.6 | 89.0 |
| Example 6 | 23.2 | 58 |
| Example 7 | 24.3 | 60.8 |
| Example 8 | 12.2 | 30.5 |
| ARCA | 9.4 | 23.5 |

As described above, specific parts of the present invention have been described in detail. It will be obvious to those skilled in the art that these specific descriptions are merely preferred exemplary embodiments and Experimental Examples, and the scope of the present invention is not limited thereby. Therefore, the substantial scope of the present invention will be defined by the appended claims and equivalents thereof.

## Claims

1. A compound represented by the following Formula 1, a stereoisomer thereof, or a salt thereof: 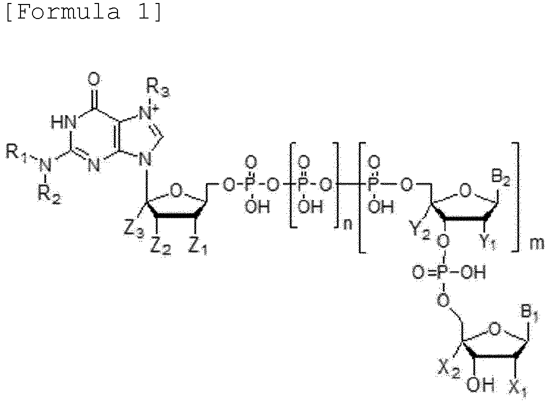 in Formula 1 above,
B₁ and B₂ are each independently a natural, unnatural or modified nucleoside base;
X₁ is -OH, -O(C₁₋₄alkyl), or -halo;
X₂ is -H or linked with X₁ to form an LNA ring {wherein at least one H of the LNA ring may be substituted with -(C₁₋₄alkyl), -OH, or -O(C₁₋₄alkyl)};
Y₁ is -O(C₁₋₄alkyl), -O(C₁₋₄alkyl)O(C₁₋₄alkyl), -CH₂O(C₁₋₄alkyl), or -halo;
Y₂ is -H or linked with Y₁ to form an LNA ring {wherein at least one H of the LNA ring may be substituted with -(C₁₋₄alkyl), -OH, or -O(C₁₋₄alkyl)};
Z₁ and Z₂ are each independently -OH, -O(C₁₋₄alkyl), - O(C₁₋₄alkyl)O(C₁₋₄alkyl), -CH₂O(C₁₋₄alkyl), or -halo;
Z₃ is -H or linked with Z₁ to form an LNA ring {wherein at least one H of the LNA ring may be substituted with -(C₁₋₄alkyl), -OH, or -O(C₁₋₄alkyl)};
n is an integer from 0 to 3;
m is an integer from 1 to 10 {wherein when m is 2 or more, each of B₂, Z₁ and Z₂ may be different from each other};
R₁ and R₂ are each independently -H or -(C₁₋₄alkyl); and
R₃ is -(C₁₋₄alkyl).

2. The compound, the stereoisomer thereof or the salt thereof according to claim 1,
wherein the unnatural or modified nucleoside base is an isomer of the natural nucleoside base or is substituted with at least one selected from the group consisting of substitution of at least one -H of the natural nucleoside base with -(C₁₋₄alkyl), -O(C₁₋₄alkyl) or -halo, substitution of at least one =CH- with =N-, and substitution of at least one =O with =S.

3. The compound, the stereoisomer thereof or the salt thereof according to claim 1, wherein
B₁ and B₂ are each independently

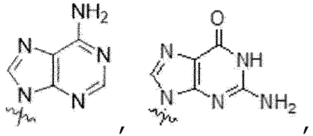

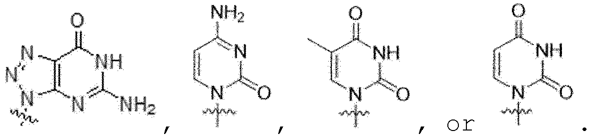

4. The compound, the stereoisomer thereof or the salt thereof according to claim 1, wherein
X₁ is -OH; and
X₂ is -H.

5. The compound, the stereoisomer thereof or the salt thereof according to claim 1, wherein
Y₁ is -O(C₁₋₄alkyl), or -O(C₁₋₄alkyl)O(C₁₋₄alkyl), or - halo; and
Y₂ is -H or linked with Y₁ to form an LNA ring {wherein at least one H of the LNA ring may be substituted with -(C₁₋₄alkyl)}.

6. The compound, the stereoisomer thereof or the salt thereof according to claim 1, wherein
Z₁ is -OH, or -O(C₁₋₄alkyl);
Z₂ is -OH; and
Z₃ is -H.

7. The compound, the stereoisomer thereof or the salt thereof according to claim 1, wherein
n is 0 or 1.

8. The compound, the stereoisomer thereof or the salt thereof according to claim 1, wherein
m is 1.

9. The compound, the stereoisomer thereof or the salt thereof according to claim 1, wherein
R₁ and R₂ are each independently -H; and
R₃ is -(C₁₋₄alkyl).

10. The compound, the stereoisomer thereof or the salt thereof according to claim 1, wherein
the compound represented by Formula 1 is selected from the group consisting of the following compounds: 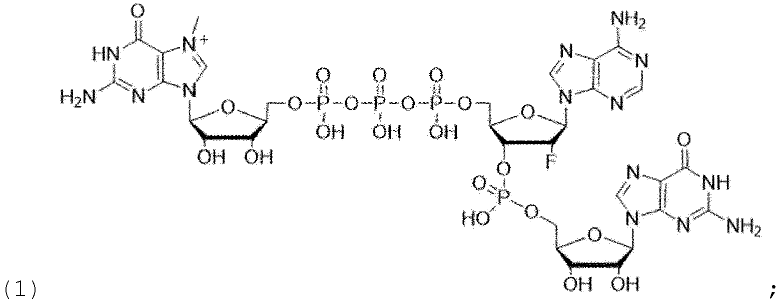 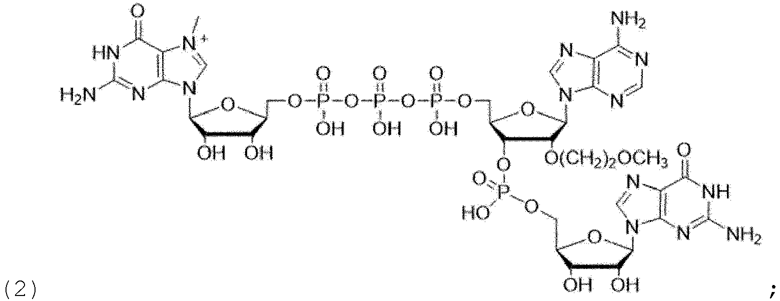 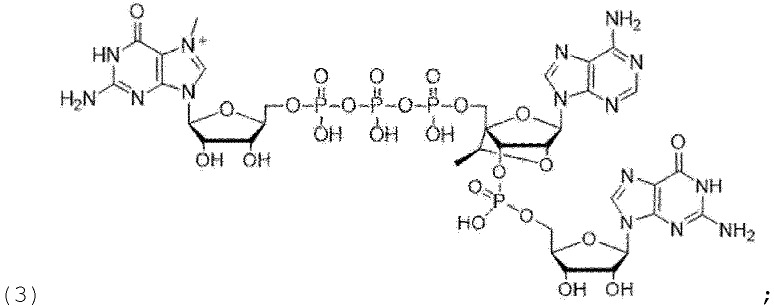 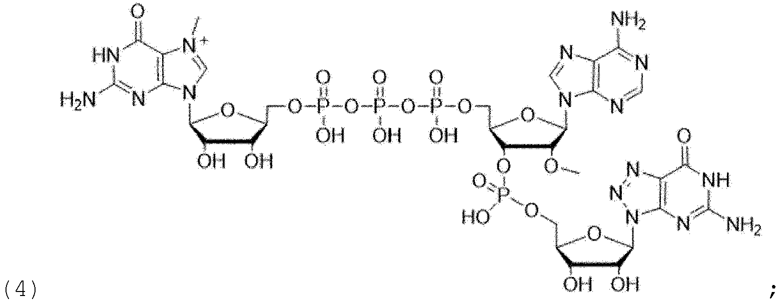 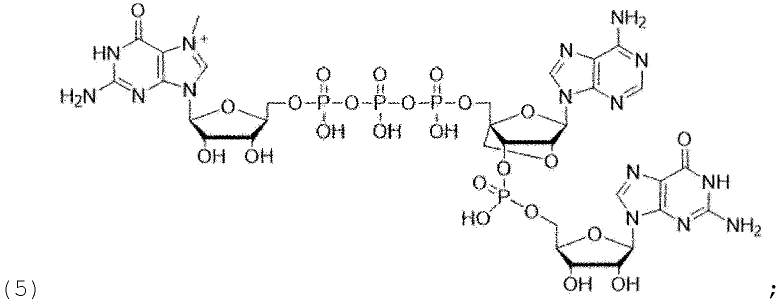 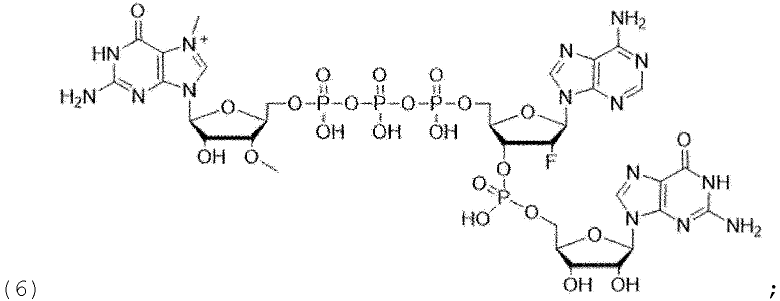 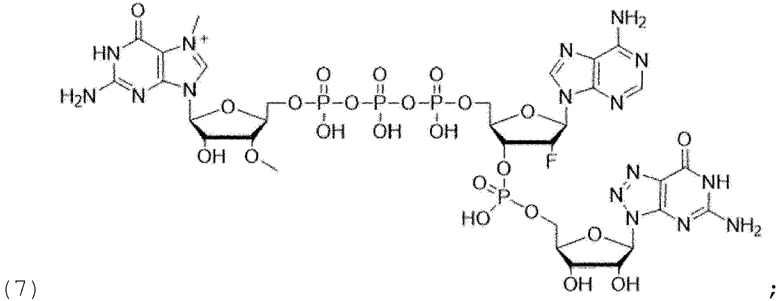 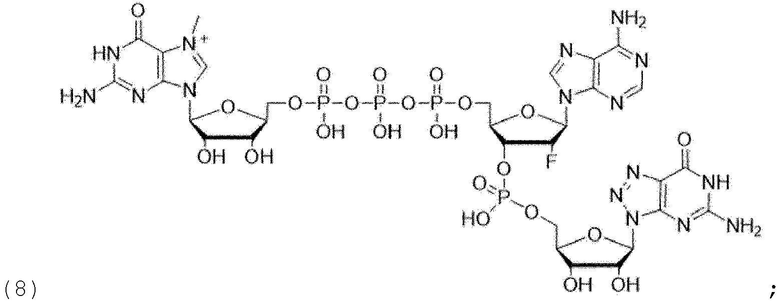 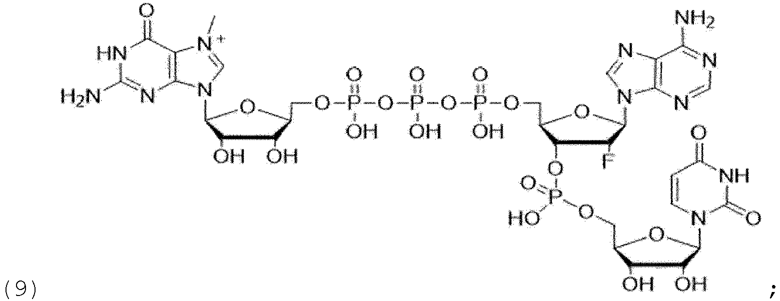 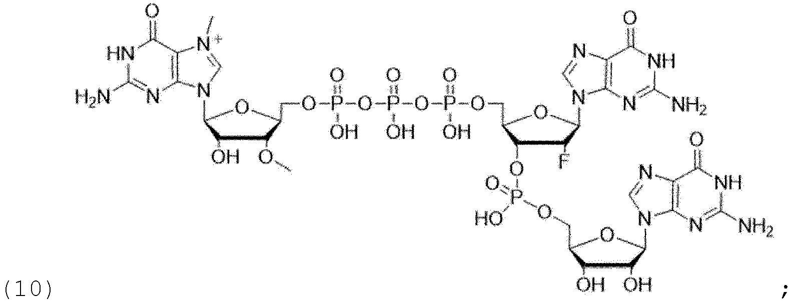 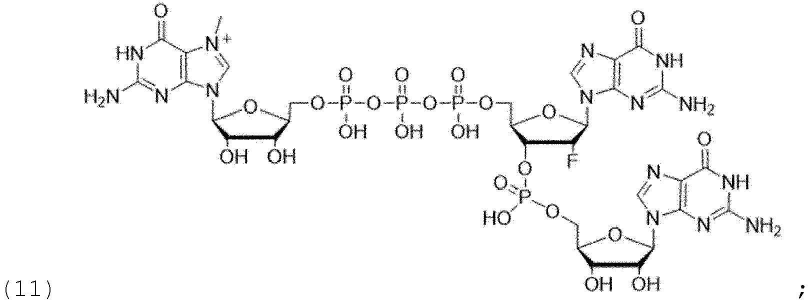 and

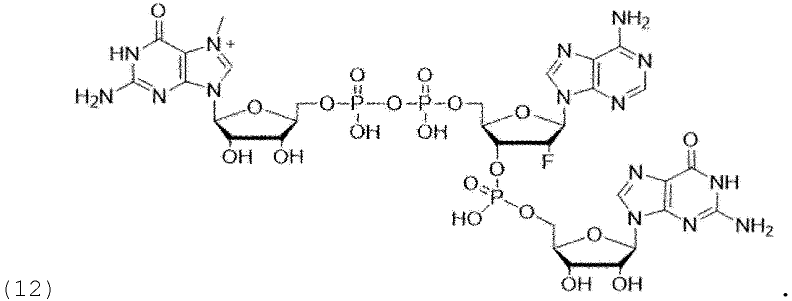

11. The compound, the stereoisomer thereof or the salt thereof according to claim 1, wherein
the compound, the stereoisomer thereof, or the salt thereof is an oligonucleotide primer for RNA capping.

12. An RNA molecule comprising the oligonucleotide primer for RNA capping according to any one of claims 1 to 11.

13. The RNA molecule according to claim 12, wherein
the oligonucleotide primer for RNA capping is attached to the 5' upstream end of the RNA molecule.

14. The RNA molecule according to claim 12, wherein
the RNA molecule is an mRNA comprising at least one coding sequence (CDS).

15. A method for synthesizing the RNA molecule according to claim 12, comprising:
(S-1) mixing a DNA template, the oligonucleotide primer for RNA capping according to any one of claims 1 to 11, and RNA polymerase; and
(S-2) performing transcription of the polynucleotide template by incubating the mixture.

16. The method for synthesizing the RNA molecule according to claim 15, wherein
the synthesizing of the RNA molecule is performed in vitro.

17. A peptide translated from the RNA molecule according to claim 12.

18. A cell into which the RNA molecule according to claim 12 is introduced.

19. A peptide produced from the cell according to claim 18.

20. A nucleic acid therapeutic agent comprising the RNA molecule according to claim 12.

21. The nucleic acid therapeutic agent according to claim 20, wherein
the therapeutic agent comprises a carrier capable of introducing the RNA molecule into a target cell.

22. A vaccine comprising the RNA molecule according to claim 12.

23. The vaccine according to claim 22, wherein
the vaccine comprises a carrier capable of introducing the RNA molecule into a target cell.

24. The vaccine according to claim 22, wherein
the vaccine is for preventing cancer or infectious disease.
